# EUROPÄISCHE PATENTANMELDUNG

(11) **EP 4 830 648 A1**
(43) Veröffentlichungstag der Anmeldung: **28.10.2026**
(21) Anmeldenummer: 25192877.6
(22) Anmeldetag: 30.07.2025
(51) Int. Cl.: A61B 17/221, A61M 29/02, A61F 2/95, A61B 17/22

(54) **DRAHTINSTRUMENT, INSBESONDERE ENDOSKOPISCHES DRAHTINSTRUMENT**

(30) Priorität: 24.04.2025 DE 102025115898
(71) Anmelder: EPflex Feinwerktechnik GmbH, 72581 Dettingen/Erms (DE)
(72) Erfinder: UIHLEIN, Bernhard, 72581 Dettingen (DE); SCHAUER, Senta, 72762 Reutlingen (DE)
(74) Vertreter: Patentanwälte Ruff, Wilhelm, Beier, Dauster & Partner mbB

(57) **Zusammenfassung**

2.1. Die Erfindung bezieht sich auf ein Drahtinstrument mit einem Kerndraht (1) und einer den Kerndraht entlang zumindest eines Teils seiner Länge umgebenden Drahthülle (2), wobei der Kerndraht gegenüber einem proximalen Hüllabschnitt (2a) der Drahthülle axial relativbeweglich angeordnet ist.

2.2. Erfindungsgemäß weist die Drahthülle einen an den proximalen Hüllabschnitt distal anschließenden Aufweitungsabschnitt (2b) auf, der biegeweicher als der proximale Hüllabschnitt ist und dafür eingerichtet ist, sich unter Einwirkung einer axialen Stauchkraft axial verkürzend von einer Ausgangsposition in eine Funktionsposition (FP) radial elastisch aufzuweiten. Dabei ist der Kerndraht mit einem distalen Endbereich (3) des Aufweitungsabschnitts axial bewegungsgekoppelt verbunden.

2.3. Verwendung z.B. für endoskopische Instrumente in der Medizintechnik.

## Beschreibung

Die Erfindung bezieht sich auf ein Drahtinstrument nach dem Oberbegriff des Anspruchs 1. Ein solches Drahtinstrument beinhaltet folglich gattungsgemäß einen Kerndraht und eine den Kerndraht entlang zumindest eines Teils seiner Länge umgebende Drahthülle, wobei der Kerndraht gegenüber einem proximalen Hüllabschnitt der Drahthülle axial relativbeweglich angeordnet ist.

Unter dem Begriff Kerndraht ist vorliegend ein beliebiges langgestrecktes Bauteil aus einem hohlen oder massiven Metall- oder Kunststoffmaterial zu verstehen, das als Kern, d.h. als eine innenliegende Komponente des Instruments fungiert. Wenn der Kerndraht aus Hohlmaterial besteht, kann das von ihm bereitgestellte Lumen optional zusätzlich genutzt werden, z.B. zum Durchführen eines Fluides oder Gases und/oder zum Durchführen bzw. Aufnehmen eines Führungsdrahtes oder eines anderen stabförmigen Körpers des Drahtinstruments oder eines anderen Instruments. Unter dem Begriff Hülle ist vorliegend allgemein ein beliebiges langgestrecktes schlauch- bzw. rohrartiges Bauteil zu verstehen, das als Umhüllung für eine andere Komponente des Instruments fungiert. Die Drahthülle ist speziell ein den Kerndraht umgebende Hülle. Bei der Hülle kann es sich je nach Anwendungsfall um ein gegenüber dem umhüllten Bauteil, wie dem Kerndraht, biegeweicheres oder biegesteiferes Schlauch- bzw. Rohrteil oder ähnliche Umhüllung aus einem Metall- oder Kunststoffmaterial handeln kann. Die Begriffe Schlauch und Rohr werden hierbei vorliegend synonym verwendet, soweit im Einzelfall nichts anderes gesagt ist.

Die Richtungsangabe distal wird synonym zur Richtungsangabe vorn verwendet, die Richtungsangabe proximal synonym zur Richtungsangabe hinten, wie beispielsweise zur Richtungsangabe bei medizinischen Instrumenten in der Endoskopietechnik üblich. Bei solchen medizinischen Instrumenten bezeichnet spezieller der distale Bereich einen im Gebrauch in einen Körperkanal oder dgl. eines menschlichen oder tierischen Körpers vorbewegten bzw. eingeführten Bereich, der proximale Bereich einen außerhalb des Körpers verbleibenden, einem Benutzer, wie einem Arzt, zur Bedienung bzw. Handhabung zugänglich bleibenden Bereich.

Drahtinstrumente der eingangs genannten Art sind beispielsweise in der endoskopischen Medizintechnik speziell in Form von Führungsdrähten und, typischerweise durch Ergänzung um ein im distalen Instrumentenbereich angeordnetes Nutzelement und meist eine oder mehrere weitere Komponenten, als Funktionsschlauchinstrumente in Form von Katheterinstrumenten gebräuchlich, wie als Steinfangkorbinstrumente mit entfaltbarem Drahtkorb als Nutzelement zum Einfangen und/oder Entfernen von Steinen, thrombotischen Blutgerinnseln und dgl. in Gewebehohlräumen, als Zangen-/Schereninstrumente z.B. für Biopsieanwendungen, als Drahtfilterinstrumente, Drahtschlingeninstrumente, Fangnetzinstrumente etc. In entsprechenden Anwendungen kann das Nutzelement auch z.B. eine optische Linse oder Kamera, eine Lichtquelle etc. sein.

Die axiale Relativbewegung zwischen dem Kerndraht und der Drahthülle bzw. dem proximalen Hüllabschnitt der Drahthülle dient zur Ausführung einer betreffenden Funktion des Instruments, z.B. dem Auffalten eines Drahtfangkorbs, dem Biegen eines distalen Führungsdrahtendes, dem Betätigen eines distalen Funktionselements, wie einer Biopsiezange oder einem Schneidmesser, etc. Um es dem Benutzer, wie einem Arzt, zu ermöglichen, die axiale Funktions-Relativbewegung zu bewirken, ist bei solchen endoskopischen Instrumenten typischerweise im proximalen Endbereich des Instruments eine entsprechende Bedieneinheit angeordnet.

Ein herkömmliches gattungsgemäßes Drahtinstrument in Form eines endoskopischen Funktionsschlauchinstruments, das z.B. als Steinfangkorbinstrument ausgeführt sein kann, ist in der Patentschrift EP 3 426 168 B1 offenbart.

In der nicht vorveröffentlichten europäischen Patentanmeldung Nr. 24151780.4 der Anmelderin ist ein endoskopisches Funktionsschlauchinstrument offenbart, bei dem es sich z.B. um ein endoskopisches Steinfangkorbinstrument handeln kann, das in einem distalen Funktionsbereich einen an der Drahthülle ausgebildeten Drahtkorb aufweist, der unter Verwendung einer speziellen Bedieneinheit durch Krümmen eines den Drahtkern und die Drahthülle umfassenden Instrumentenkörpers in einem proximalen Bedienbereich radial aufgeweitet werden kann. Die Bedieneinheit eignet sich besonders auch für ein präzises, feinfühliges Einstellen vergleichsweise kleiner Axialhubamplituden.

Der Erfindung liegt als technisches Problem die Bereitstellung eines Drahtinstruments der eingangs genannten Art zugrunde, das gegenüber dem oben erwähnten Stand der Technik insbesondere fertigungstechnische und/oder funktionelle Vorteile bietet.

Die Erfindung löst dieses Problem durch die Bereitstellung eines Funktionsschlauchinstruments mit den Merkmalen des Anspruchs 1. Vorteilhafte Weiterbildungen der Erfindung sind Gegenstand der Unteransprüche, deren Wortlaut hiermit durch Verweis zum Bestandteil der Beschreibung gemacht wird. Dies schließt insbesondere auch alle Ausführungsformen der Erfindung ein, die sich aus den Merkmalskombinationen ergeben, die durch die Rückbezüge in den Unteransprüchen definiert sind.

Beim erfindungsgemäßen Drahtinstrument weist die Drahthülle einen an den proximalen Hüllabschnitt distal anschließenden Aufweitungsabschnitt auf, der biegeweicher als der proximale Hüllabschnitt ist und dafür eingerichtet ist, sich unter Einwirkung einer axialen Stauchkraft axial verkürzend von einer Ausgangsposition in eine Funktionsposition radial elastisch aufzuweiten. Dabei ist der Kerndraht mit einem distalen Endbereich des Aufweitungsabschnitts axial bewegungsgekoppelt verbunden.

Folglich kann bei dem erfindungsgemäßen Drahtinstrument durch das Ausüben der axialen Stauchkraft auf den Aufweitungsabschnitt, d.h. einer axial stauchend auf die den Aufweitungsabschnitt wirkenden Druckkraft, die Drahthülle in ihrem Aufweitungsabschnitt radial elastisch aufgeweitet werden. Da der proximale Hüllabschnitt biegesteifer ist als der Aufweitungsabschnitt, kann sich der Aufweitungsabschnitt für das radialen Aufweiten am proximalen Hüllabschnitt abstützen, während der proximale Hüllabschnitt zumindest weitestgehend formstabil bleibt. Da die Aufweitung elastisch ist, kann die Drahthülle in ihrem Aufweitungsabschnitt selbsttätig wieder von der radial aufgeweiteten Funktionsposition in die nicht aufgeweitete Ausgangsposition zurückkehren, wenn die axiale Stauchkraft nicht mehr einwirkt.

Die Stauchkraft kann vom Benutzer insbesondere durch eine entsprechende, stauchende Axialbewegung des Kerndrahts relativ zum proximalen Hüllabschnitt erzeugt werden, im Allgemeinen eine relativ zum proximalen Hüllabschnitt nach hinten gerichtete Axialbewegung des Kerndrahts. Da der Kerndraht mit dem distalen Endbereich des Aufweitungsabschnitts axial bewegungsgekoppelt verbunden ist, überträgt sich die vom Benutzer über den Kerndraht eingeleitete axiale Stauchkraft auf den Aufweitungsabschnitt und bewirkt dessen axiale Stauchung, i.e. Verkürzung, nebst der gleichzeitigen radialen Aufweitung.

Der aufgeweitete Funktionszustand des Aufweitungsabschnitts lässt sich für eine entsprechende Funktion nutzen, beispielsweise zum kraftschlüssigen und/oder formschlüssigen, bewegungskoppelnden Verbinden der Drahthülle mit einer umgebenden Komponente des Instruments, wie einem Instrumentenschlauch. Letzterer kann beispielsweise ein Katheterschlauch eines entsprechenden Katheterinstruments sein. Des Weiteren lässt sich die radiale Aufweitung beispielsweise zum Dehnen oder Erweitern von Engstellen in Körpergewebekanälen eines menschlichen oder tierischen Körpers, i.e. Stenosen, verwenden. Auch zum Aufdehnen bzw. Auffalten von Stents zum Einsetzen in Stenosen kann die radiale Aufweitung der Drahthülle in ihrem Aufweitungsabschnitt genutzt werden.

Mit dem proximalen Hüllabschnitt ist vorliegend der an den Aufweitungsabschnitt proximal anschließende Teil der Drahthülle zu verstehen. Bevorzugt befindet sich der Aufweitungsabschnitt in einem distalen Bereich bzw. distalen Endbereich der Drahthülle, d.h. mit deutlich geringerem Abstand zu dessen distalem Ende als zu dessen proximalem Ende. Der proximale Hüllabschnitt der Drahthülle erstreckt sich in diesem Fall bis weit in den distalen Bereich der Drahthülle. Ein ggf. distal verbleibender Teil der Drahthülle, d.h. ein distaler Hüllabschnitt derselben, vom Aufweitungsabschnitt bis zum distalen Ende der Drahthülle besitzt dann eine deutlich geringere Länge als der proximale Hüllabschnitt.

In einer Weiterbildung der Erfindung beinhaltet die Drahthülle einen an den Aufweitungsabschnitt distal anschließenden distalen Hüllabschnitt, der biegesteifer als der Aufweitungsabschnitt ist, wobei der Kerndraht mit dem distalen Hüllabschnitt axial bewegungsgekoppelt verbunden ist. In dieser Ausführung des Drahtinstruments ist der Aufweitungsabschnitt zwischen den beiden biegesteiferen Hüllabschnitten, d.h. dem distalen Hüllabschnitt und dem proximalen Hüllabschnitt, angeordnet. Da der Kerndraht mit dem distalen Hüllabschnitt axial bewegungsgekoppelt verbunden ist, überträgt sich die durch die stauchende Axialbewegung des Kerndrahts erzeugte axiale Stauchkraft über den distalen Hüllabschnitt auf den Aufweitungsabschnitt der Drahthülle. In alternativen Ausführungen kann der Kerndraht an einem distalen Kerndraht-Endbereich direkt mit dem distalen Endbereich des Aufweitungsabschnitts verbunden sein.

In einer Weiterbildung der Erfindung umfasst der Aufweitungsabschnitt mehrere axial hintereinanderliegende Aufweitungsteilabschnitte, von denen jeder biegeweicher als der proximale Hüllabschnitt ist und dafür eingerichtet ist, sich unter Einwirkung der axialen Stauchkraft radial elastisch aufzuweiten. Diese Ausführung ermöglicht ein radiales Aufweiten der Drahthülle in ihrem Aufweitungsabschnitt in mehreren axial voneinander beabstandeten Aufweitungszonen, indem sich die Aufweitungsteilabschnitte einzeln unter Einwirkung der axialen Stauchkraft radial elastisch aufweiten.

In einer Weiterbildung der Erfindung beinhaltet der Aufweitungsabschnitt einen fluiddichten Abschnitt der Drahthülle. In dieser Ausführung lässt sich das Drahtinstrument bei Bedarf für eine Abdichtungsfunktion nutzen, beispielsweise um ein Fluid innerhalb der Drahthülle oder außerhalb der Drahthülle zu führen, ohne dass das Fluid radial durch den Aufweitungsabschnitt hindurchtritt, oder um einen radial an die Drahthülle außen angrenzenden Ringraum in axialer Richtung abzudichten. In letzterem Fall trennt der Aufweitungsabschnitt in seiner radial aufgeweiteten Funktionsposition einen distalen Teil des die Drahthülle umgebenden Ringraums fluiddicht von einem proximalen Teil des Ringraums.

In einer Weiterbildung der Erfindung beinhaltet der Aufweitungsabschnitt einen perforierten Abschnitt der Drahthülle. Diese Ausführung des Drahtinstruments ermöglicht bei Bedarf einen Fluiddurchtritt in radialer Richtung durch den Aufweitungsabschnitt hindurch. Zudem kann die Ausführung fertigungstechnische Vorteile bieten, indem der perforierte Aufweitungsabschnitt relativ einfach beispielsweise durch Laserschneiden aus einem Vollwandmaterial gebildet werden kann. Die durch das Perforieren verursachte Materialschwächung schafft in diesem Fall die Voraussetzung, dass sich der Aufweitungsabschnitt unter Einwirkung der axialen Stauchkraft, d.h. Druckkraft, in gewünschter Weise radial elastisch aufweitet.

In einer Weiterbildung der Erfindung ist der Aufweitungsabschnitt einteilig mit dem proximalen Hüllabschnitt und/oder mit dem distalen Hüllabschnitt gebildet. Die bei dieser Ausführung mögliche einteilige Fertigung des Aufweitungsabschnitts zusammen mit dem proximalen und/oder dem distalen Hüllabschnitt kann dazu beitragen, den Fertigungsaufwand möglichst gering zu halten.

In einer anderen Weiterbildung der Erfindung ist der Aufweitungsabschnitt durch ein separates Aufweitungshülsenstück gebildet. Das Aufweitungshülsenstück ist an einem proximalen Endbereich mit einem distalen Endbereich des proximalen Hüllabschnitts direkt oder über ein Verbindungshülsenstück verbunden und/oder an dem distalen Endbereich mit einem proximalen Endbereich des distalen Hüllabschnitts direkt oder über ein Verbindungshülsenstück verbunden. In dieser Ausführung ist der Aufweitungsabschnitt durch das Aufweitungshülsenstück als ein separates Bauteil realisiert. Damit kann der Aufweitungsabschnitt, wenn gewünscht, materialtechnisch bzw. im Hinblick auf die physikalischen und chemischen Eigenschaften seines Materials vom Material des proximalen bzw. distalen Hüllabschnitts der Drahthülle und dessen physikalischen und chemischen Eigenschaften entkoppelt werden.

Das Verbindungshülsenstück kann aus einem Metallmaterial oder einem Kunststoffmaterial bestehen. Beispielsweise können das oder die Verbindungshülsenstücke aus einem Kunststoffmaterial bestehen, das härterer bzw. biegesteifer oder weniger elastisch ist als ein Kunststoffmaterial, aus dem das Aufweitungshülsenstück gebildet sein kann. Diese Ausführung begünstigt ein festes und zuverlässiges Verbinden von Aufweitungshülsenstück und jeweiligem Verbindungshülsenstück durch Verschmelzen der betreffenden Kunststoffmaterialien miteinander bzw. aneinander. Zudem schützt diese Ausführung davor, dass sich das elastischere, weichere Material des Aufweitungshülsenstücks unter Druckbelastung im Betrieb eventuell in unerwünschter Weise über das angrenzende härtere Material des jeweiligen Verbindungshülsenstücks stülpt. Alternativ kann die Verbindung von Aufweitungshülsenstück und jeweiligem Verbindungshülsenstück z.B. durch eine Klebeverbindung realisiert sein.

In einer Weiterbildung der Erfindung ist der Aufweitungsabschnitt mehrlagig aufgebaut, wobei er zumindest eine Innenlage, d.h. eine am weitesten radial innen gelegene Lage, und eine Außenlage, d.h. eine am weitesten radial außen gelegene Lage, aufweist. Dabei unterscheiden sich zumindest zwei der Lagen in ihrem Material und/oder ihrer Struktur und/oder ihren physikalischen Eigenschaften. Diese Ausführung ermöglicht es, den Aufweitungsabschnitt der Drahthülle spezifisch auf geforderte bzw. gewünschte Eigenschaften auszulegen. Beispielsweise können sich Innenlage und Außenlage im Material oder ihrer Struktur oder ihren physikalischen Eigenschaften unterscheiden. So kann z.B. die Außenlage andere Gleiteigenschaften aufweisen als die Innenlage, oder es wird für die eine Lage eine fluiddichte Ausführung und für die andere Lage eine fluiddurchlässige, z.B. gewebte oder anderweitig perforierte, Struktur gewählt. Auch kann es sich auf Wunsch bei der einen Lage um ein Kunststoffmaterial und bei der anderen Lage um ein Metallmaterial handeln.

In einer Ausgestaltung der Erfindung sind die Innenlage und die Außenlage jeweils fluiddicht ausgeführt, und der Aufweitungsabschnitt weist zusätzlich eine Zwischenlage aus einem flüssigen oder gelartigen Material zwischen der Innenlage und der Außenlage auf. In dieser Ausführung ist die Zwischenlage aus flüssigem bzw. gelartigem Material zwischen die Innenlage und die Außenlage eingebettet. Die Verwendung des flüssigen bzw. gelartigen Materials kann für entsprechende Anwendungen die elastischen Aufweitungseigenschaften begünstigen. Zudem kann sich das fließfähige Material dicht und eng auch gegen komplexere äußere Strukturen anlegen, da einwirkende Druckkräfte gleichmäßig über das Materialvolumen verteilt werden. Das flüssige bzw. gelartige Material eignet sich auch gut für eine Beimischung von Additiven für entsprechende Zwecke, z.B. von Markerpartikeln zur verbesserten Sichtbarkeit bzw. Lokalisierung unter Röntgenstrahlung oder unter Magnetresonanzbedingungen.

In einer Ausgestaltung der Erfindung ist die Außenlage von einem fluiddichten Hüllschlauch gebildet. Der Hüllschlauch erstreckt sich nur über die Länge der Innenlage hinweg oder alternativ über die Länge der Innenlage und über zumindest einen Teil der Länge des proximalen Hüllabschnitts und/oder des distalen Hüllabschnitts hinweg. Dies stellt eine spezifische Ausführung für einen fluiddichten Aufweitungsabschnitt dar. Der Hüllschlauch lässt sich relativ einfach fertigen und anbringen, und er kann bei Bedarf nicht nur auf dem Aufweitungsabschnitt angebracht werden, sondern auch darüber hinaus auf der gesamten Drahthülle oder zumindest bereichsweise auf deren proximalem Hüllabschnitt bzw. deren distalem Hüllabschnitt.

In einer Weiterbildung der Erfindung enthält der Aufweitungsabschnitt und/oder der proximale Hüllabschnitt und/oder der distale Hüllabschnitt und/oder das Verbindungshülsenstück ein Röntgenstrahlungs-sichtbares und/oder ein Magnetresonanz(MR)-sichtbares Markermaterial. Diese Maßnahme ist für Anwendungen des Drahtinstruments nützlich, bei denen eine Sichtbarkeit des Instruments bzw. von Teilen des Instruments unter Röntgenstrahlung oder unter Magnetresonanzbedingungen gefordert bzw. gewünscht ist. Geeignete Markermaterialien für Röntgensichtbarkeit bzw. MR-Sichtbarkeit sind dem Fachmann geläufig, wobei vorliegend ein beliebiges derartiges Material verwendet werden kann.

In einer Weiterbildung der Erfindung ist der Kerndraht über eine distale Endkuppe direkt mit dem distalen Endbereich des Aufweitungsabschnitts oder mit einem distalen Endbereich des distalen Hüllabschnitts verbunden. In dieser Ausführung des Drahtinstruments schließt der Kerndraht am distalen Ende mit der Endkuppe ab, die üblicherweise eine abgerundete, z.B. halbkugelige, Form besitzt, um Gewebeverletzungen vorzubeugen, was daher auch als atraumatische Form bezeichnet wird. Im vorliegenden Fall dient die distale Endkuppe des Kerndrahts zudem als Verbindungselement zum Aufweitungsabschnitt oder zum distalen Hüllabschnitt.

In einer Weiterbildung der Erfindung ist der Kerndraht über eine umfangsseitige Verbindung direkt mit dem distalen Endbereich des Aufweitungsabschnitts oder mit einem umgebenden Bereich des distalen Hüllabschnitts verbunden. In dieser Ausführung erfolgt die Ankopplung des Kerndrahts und damit die Übertragung der axialen Stauchkraft vom Kerndraht auf den Aufweitungsabschnitt über die umfangsseitige Verbindung des Kerndrahts direkt mit dem Aufweitungsabschnitt oder mit dem distalen Hüllabschnitt.

In einer Weiterbildung der Erfindung ist der proximale Hüllabschnitt zumindest bereichsweise durch ein Schraubenfederstück oder ein Hülsenstück aus massivem Hülsenwandmaterial gebildet. Dies stellt je nach Anwendungsfall günstige Realisierungen für den proximalen Hüllabschnitt dar, wie sie an sich für Drahthüllen in der Endoskopietechnik gebräuchlich sind.

In einer Weiterbildung der Erfindung ist der distale Hüllabschnitt zumindest bereichsweise durch ein Schraubenfederstück oder ein Hülsenstück aus massivem Hülsenwandmaterial gebildet. Analog wie zuvor für den proximalen Hüllabschnitt erwähnt, stellt dies für bestimmte Anwendungen günstige Realisierungen für den distalen Hüllabschnitt dar.

In einer Weiterbildung der Erfindung bilden der Kerndraht und die Drahthülle einen endoskopischen Führungsdraht. Dies stellt eine vorteilhafte Anwendung des Drahtinstruments dar. Der in seinem Aufweitungsabschnitt radial aufweitbare, i.e. radial expandierbare, Führungsdraht kann beispielsweise dazu genutzt werden, an ihm einen äußeren Instrumentenschlauch, wie einen Katheterschlauch, axial bewegungsgekoppelt festzuklemmen oder den Instrumentenschlauch proximal nach hinten abzudichten oder in einem Körpergewebekanal eine Stenose oder einen Stent aufzudehnen bzw. aufzuweiten.

In einer Weiterbildung der Erfindung ist der Kerndraht als Hohldraht aus einem hohlen Metall- oder Kunststoffmaterial gebildet. In diesem Fall stellt der Kerndraht durch sein hohles Inneres ein Lumen zur Verfügung, das bei Bedarf zusätzlich genutzt werden kann, z.B. dazu, ein Fluid oder ein Gas hindurchzuleiten und/oder einen Führungsdraht aufzunehmen, über den dann das Drahtinstrument geschoben werden kann. Alternativ ist der Kerndraht aus einem Vollmaterial aus Metall und/oder Kunststoff gebildet.

In einer Weiterbildung der Erfindung beinhaltet das Drahtinstrument eine proximale, nutzerbetätigbare Bedieneinheit zur axialen Verschiebung des Kerndrahtes gegenüber dem proximalen Hüllabschnitt. Durch entsprechende Ausführung der Bedieneinheit ermöglicht dies eine optimale Bedienfunktionalität für den Benutzer, um die Drahthülle mit ihrem Aufweitungsabschnitt zwischen ihrer Ausgangsposition und ihrer Funktionsposition zu verlagern. Als Bedieneinheit kann jede geeignete herkömmliche Bedieneinheit verwendet werden, insbesondere die Bedieneinheiten gemäß dem oben erwähnten Stand der Technik, wie gemäß der Patentschrift EP 3 426 168 B1 und der europäischen Patentanmeldung Nr. 24151780.4.

In einer Ausgestaltung der Erfindung beinhaltet das Drahtinstrument einen Instrumentenschlauch, der den Kerndraht und die Drahthülle wenigstens im Bereich des proximalen Hüllabschnitts und des Aufweitungsabschnitts umgibt. Dabei umgibt der Instrumentenschlauch den proximalen Hüllabschnitt mit radialem Abstand. Ebenso umgibt der Instrumentenschlauch den Aufweitungsabschnitt mit radialem Abstand, wenn sich der Aufweitungsabschnitt in seiner nicht aufgeweiteten Ausgangsposition befindet. Wenn sich der Aufweitungsabschnitt in seiner aufgeweiteten Funktionsposition befindet, legt sich der Aufweitungsabschnitt axial bewegungskoppelnd radial gegen den Instrumentenschlauch an. Zudem kann optional eine Abdichtfunktion bereitgestellt werden, indem der Aufweitungsabschnitt bei entsprechender Ausführung in seiner aufgeweiteten Funktionsposition den Ringraum zwischen Drahthülle und Instrumentenschlauch fluiddicht abschließt.

In einer weiteren Ausgestaltung der Erfindung ist das Drahtinstrument ein endoskopisches Katheterinstrument, wobei der Instrumentenschlauch einen Katheterschlauch bildet. Dies stellt eine weitere vorteilhafte Anwendung des Drahtinstruments dar. Der Aufweitungsabschnitt kann hierbei z.B. dazu genutzt werden, in seiner aufgeweiteten Funktionsposition den Katheterschlauch axial bewegungsfest an die als Führungsdraht fungierende Einheit aus Kerndraht und Drahthülle zu koppeln, um den Katheterschlauch zusammen mit dem Führungsdraht in eine Körpergewebekanal einzuführen bzw. vorzuschieben. Das vermeidet, dass sich der Katheterschlauch beim Aufschieben auf den Führungsdraht, wenn dieser vorab eingeschoben wird, aufstaut, wenn er mit seinem Vorderende hängen bleibt, und Falten bildet. Weiter kann der Aufweitungsabschnitt bei entsprechendem Bedarf und entsprechender Ausführung dazu genutzt werden, den Ringraum im Katheterschlauch um den Führungsdraht herum proximal, d.h. in Richtung nach hinten, abzudichten, so dass keine Gewebeflüssigkeiten oder dgl. in den proximalen Instrumentenbereich gelangen.

Vorteilhafte Ausführungsformen der Erfindung sind in den Zeichnungen dargestellt. Diese und weitere Ausführungsformen der Erfindung werden nachfolgend näher erläutert. Hierbei zeigen:
- Fig. 1: eine Längsschnittansicht eines vorderen Teils einer z.B. als endoskopischer Führungsdraht verwendbaren Ausführung des erfindungsgemäßen Drahtinstruments mit dem Aufweitungsabschnitt in der Ausgangsposition,
- Fig. 2: die Ansicht von Fig. 1 mit dem Aufweitungsabschnitt in der aufgeweiteten Funktionsposition,
- Fig. 3: die Ansicht von Fig. 2 für eine weitere, z.B. als Führungsdraht verwendbare Ausführung des erfindungsgemäßen Drahtinstruments mit einem mehrteiligem Aufweitungsabschnitt,
- Fig. 4: eine Seitenansicht eines vorderen Teils einer weiteren, z.B. als Führungsdraht verwendbaren Ausführung des erfindungsgemäßen Drahtinstruments mit einem perforierten Aufweitungsabschnitt in der Ausgangsposition,
- Fig. 5: eine Seitenansicht eines vorderen Teils einer weiteren, z.B. als Führungsdraht verwendbaren Ausführung des erfindungsgemäßen Drahtinstruments mit einem perforierten Aufweitungsabschnitt als separates Aufweitungshülsenstück in der Ausgangsposition,
- Fig. 6: eine Längsschnittansicht eines vorderen Teils einer weiteren, z.B. als Führungsdraht verwendbaren Ausführung des erfindungsgemäßen Drahtinstruments mit einem mehrlagigen Aufweitungsabschnitt mit fluiddichter Außenlage in der Ausgangsposition,
- Fig. 7: eine Seitenansicht eines vorderen Teils einer weiteren, z.B. als Führungsdraht verwendbaren Ausführung des erfindungsgemäßen Drahtinstruments mit einem mehrlagigen Aufweitungsabschnitt mit Hüllschlauch in der Ausgangsposition,
- Fig. 8: die Ansicht von Fig. 3 für eine Variante mit modifiziertem Aufbau eines distalen Hüllabschnitts der Drahthülle,
- Fig. 9: die Ansicht von Fig. 1 für eine Variante mit mehrlagigem Aufweitungsabschnitt,
- Fig. 10: eine Längsschnittansicht einer Variante der Ausführung von Fig. 5 mit Schraubenfederstücken für die Drahthülle,
- Fig. 11: eine Seitenansicht eines vorderen Teils einer weiteren, z.B. als Führungsdraht verwendbaren Ausführung des erfindungsgemäßen Drahtinstruments mit einem mehrlagigen Aufweitungsabschnitt mit fluider oder gelartiger Zwischenlage in der Ausgangsposition,
- Fig. 12: eine Detailansicht eines Bereichs **XII** von Fig. 11,
- Fig. 13: die Ansicht von Fig. 8 für eine Variante bzgl. des Aufbaus des Aufweitungsabschnitts und des proximalen Hüllabschnitts der Drahthülle,
- Fig. 14: die Ansicht von Fig. 1 für eine z.B. als Katheterinstrument verwendbare Erweiterung des erfindungsgemäßen Drahtinstruments um einen äußeren Instrumentenschlauch,
- Fig. 15: die Ansicht von Fig. 2 für das erweiterte Drahtinstrument von Fig. 14,
- Fig. 16: eine schematische Längsschnittansicht des Drahtinstruments von Fig. 15 in einer beispielhaften Gebrauchssituation,
- Fig. 17: die Ansicht von Fig. 3 für eine z.B. als Katheterinstrument verwendbare Erweiterung des erfindungsgemäßen Drahtinstruments um einen äußeren Instrumentenschlauch,
- Fig. 18: die Längsschnittansicht von Fig. 13 in einer schematisch angedeuteten Gebrauchssituation des Drahtinstruments in einem Körpergewebekanal,
- Fig. 19: die Ansicht von Fig. 9 für eine Variante mit einem Hohlrohr als Kerndraht,
- Fig. 20: die Ansicht von Fig. 11 für eine Variante mit einem Hohlrohr als Kerndraht und mit separatem Aufweitungshülsenstück und zugeordneten Verbindungshülsenstücken aus Kunststoffmaterial und
- Fig. 21: eine Detailansicht eines Bereichs XXI von Fig. 20.

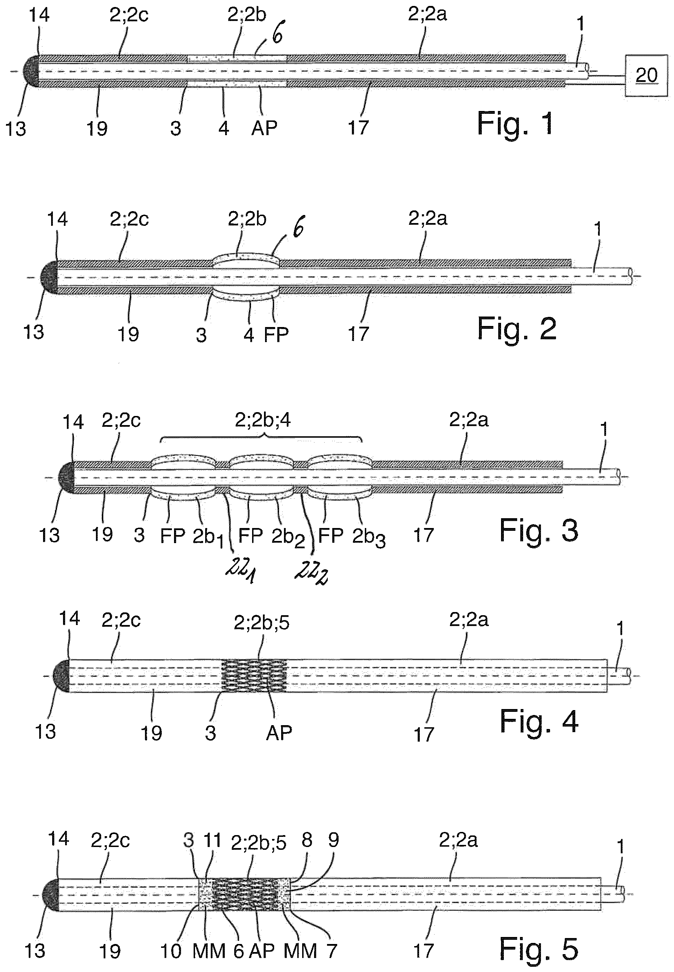

Wie in den Fig. 1 bis 21 in verschiedenen Ausführungen und Ansichten veranschaulicht, umfasst das erfindungsgemäße Drahtinstrument einen Kerndraht 1 und eine Drahthülle 2, die den Kerndraht 1 entlang zumindest eines Teils seiner Länge umgibt. Der Kerndraht ist 1 gegenüber einem proximalen Hüllabschnitt 2a der Drahthülle 2 axial relativbeweglich angeordnet.

Die Drahthülle 2 weist einen an den proximalen Hüllabschnitt 2a distal anschließenden Aufweitungsabschnitt 2b auf, der biegeweicher als der proximale Hüllabschnitt 2a ist. Der Aufweitungsabschnitt 2b ist dafür eingerichtet, sich unter Einwirkung einer axialen Stauchkraft axial verkürzend von einer Ausgangsposition AP in eine Funktionsposition FP radial elastisch aufzuweiten. Der Kerndraht 1 ist mit einem distalen Endbereich 3 des Aufweitungsabschnitts 2b axial bewegungsgekoppelt verbunden.

Unter der axial bewegungsgekoppelten Verbindung ist zu verstehen, dass sich der distale Endbereich 3 des Aufweitungsabschnitts 2b mit dem Kerndraht 1 mitbewegt, wenn der Kerndraht 1 relativ zum proximalen Hüllabschnitt 2a der Drahthülle 2 axial bewegt wird. Wie oben erläutert, dient diese axiale Relativbewegung des Kerndrahts 1 gegenüber dem proximalen Hüllabschnitt 2a der Drahthülle 2 zur Bereitstellung der axialen Stauchkraft auf den Aufweitungsabschnitt 2b. Dazu wird die vom Benutzer durch entsprechendes Bedienen des Drahtinstruments bewirkte axiale Relativbewegung des Kerndrahts 1 auf den mit seinem distalen Endbereich 3 an den Kerndraht 1 angekoppelten Aufweitungsabschnitt 2b übertragen, wodurch dieser die entsprechende Stauchkraft erfährt.

In den Figuren befindet sich der proximale Teil des Drahtinstruments jeweils auf der rechten Seite, der distale Bereich des Drahtinstruments auf der linken Seite. Zum Erzeugen der Stauchkraft auf den Aufweitungsabschnitt 2b zieht der Benutzer den Kerndraht 1 proximal nach hinten, d.h. in den Figuren nach rechts, und/oder schiebt den proximalen Hüllabschnitt 2a der Drahthülle 2 distal nach vorn, d.h. in den Figuren nach links. Dies führt zur axialen Verkürzung und gleichzeitigen elastischen radialen Aufweitung des Aufweitungsabschnitts 2b, der sich dadurch aus seiner nicht aufgeweiteten Ausgangsposition AP heraus in seine expandierte, radial aufgeweitete Funktionsposition FP verformt. Dies ist anschaulich im Vergleich in den Figuren 1 und 2 für die dortige Instrumentenausführung veranschaulicht.

In vorteilhaften Ausführungsformen beinhaltet die Drahthülle 2 einen an den Aufweitungsabschnitt 2b distal anschließenden distalen Hüllabschnitt 2c, der biegesteifer als der Aufweitungsabschnitt 2b ist und mit dem der Kerndraht 1 axial bewegungsgekoppelt verbunden ist. Die in den Figuren gezeigten Ausführungen sind sämtlich von dieser Bauart. In alternativen, nicht gezeigten Ausführungen schließt der Kerndraht 1 mit seinem distalen Endbereich direkt an den distalen Endbereich 3 des Aufweitungsabschnitts 2b an.

In entsprechenden Realisierungen umfasst der Aufweitungsabschnitt 2b mehrere axial hintereinanderliegende Aufweitungsteilabschnitte, von denen jeder biegeweicher als der proximale Hüllabschnitt 2a ist und jeder dafür eingerichtet ist, sich unter Einwirkung der axialen Stauchkraft radial elastisch aufzuweiten. Die Figuren 3, 8 und 17 zeigen zugehörige Beispiele, bei denen jeweils drei Aufweitungsteilabschnitte 2b₁, 2b₂, 2b₃ axial hintereinanderliegend und mit etwas axialem Abstand voneinander vorgesehen sind. Der Aufweitungsabschnitt 2b ist hierbei jeweils in seiner radial aufgeweiteten Funktionsposition FP gezeigt. Wie daraus ersichtlich, weitet sich jeder der Aufweitungsabschnitte 2b₁, 2b₂, 2b₃ einzeln auf, so dass entlang der axialen Ausdehnung des Aufweitungsabschnitts 2b mehrere, hier beispielhaft drei, radial aufgeweitete Zonen gebildet sind. Die Aufweitungsteilabschnitte 2b₁, 2b₂, 2b₃ sind in entsprechenden Ausführungen, wie in den gezeigten Fällen, über Abstandhülsenstücke 22₁, 22₂ miteinander verbunden, die den Kerndraht 1 axial beweglich umgeben und beispielsweise aus dem Material des proximalen Hüllabschnitts 2a oder dem Material des distalen Hüllabschnitts 2c gebildet sind.

In entsprechenden Ausführungen beinhaltet der Aufweitungsabschnitt 2b einen fluiddichten Abschnitt 4 der Drahthülle 2, was bedeuten kann, dass der Aufweitungsabschnitt 2b zumindest bereichsweise und vorzugsweise insgesamt fluiddicht bereitgestellt ist, wie dies in den Ausführungsbeispielen der Figuren 1 bis 3, 6 bis 9 und 11 bis 18 der Fall ist. In dieser Ausführung kann das Drahtinstrument eine Abdichtfunktion erfüllen.

In entsprechenden Ausführungen beinhaltet der Aufweitungsabschnitt 2b einen perforierten, d.h. fluiddurchlässigen, Abschnitt 5 der Drahthülle 2, wie dies bei den Ausführungsbeispielen der Figuren 4, 5 und 10 der Fall ist. Der perforierte Abschnitt 5 kann z.B. ein durch Laserschneiden bearbeiteter Abschnitt eines Vollwandmaterials gebildet sein, aus dem die Drahthülle 2 z.B. in ihrem proximalen Hüllabschnitt 2a bestehen kann. Diese Herstellung einer perforierten Struktur ist dem Fachmann z.B. aus der entsprechenden Fertigung von Stents und Filterstrukturen für endoskopische Anwendungen geläufig. Alternativ kann der perforierte Abschnitt 5 eine separat von der übrigen Drahthülle 2 gebildete und an der Drahthülle 2 angebrachte Struktur sein, wie eine Gewebestruktur.

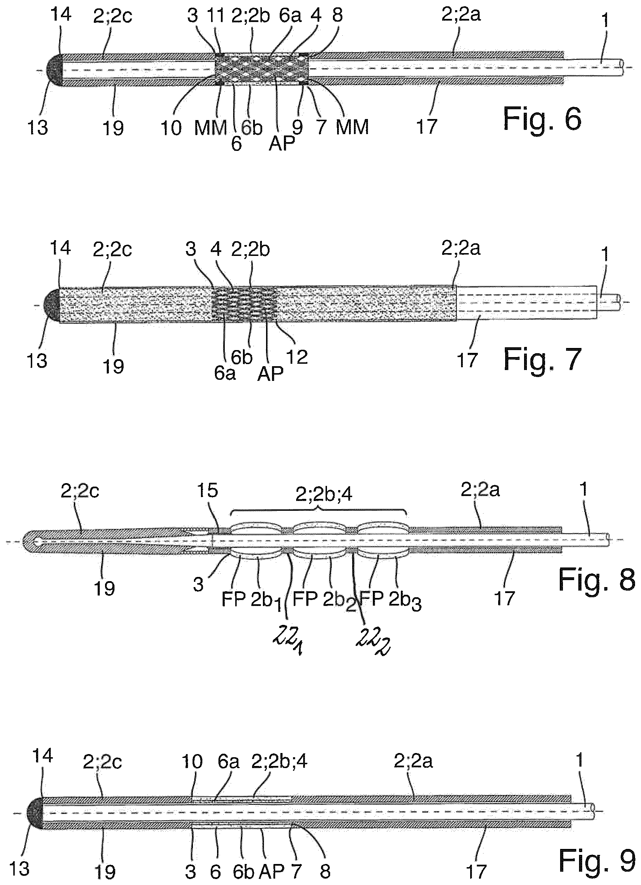

In entsprechenden Realisierungen ist der Aufweitungsabschnitt 2b einteilig mit dem proximalen Hüllabschnitt 2a und/oder mit dem distalen Hüllabschnitt 2c gebildet, wie dies beispielsweise für die Ausführung von Fig. 4 möglich ist. In entsprechenden Ausführungsvarianten ist zumindest eine von mehreren Komponenten bzw. Lagen des Aufweitungsabschnitts 2b einteilig mit dem proximalen Hüllabschnitt 2a und/oder mit dem distalen Hüllabschnitt 2c gebildet, wie dies bei der Ausführung der Fig. 7 der Fall sein kann. Eine einteilige Fertigung des Aufweitungsabschnitts 2b ist auch für Ausführungen möglich, die denjenigen der Fig. 1 bis 3 mit dem Unterschied entsprechen, dass der Aufweitungsabschnitt 2b durch Einbringen axialer Schlitze in die Drahthülle 2 gebildet ist und dadurch einen perforierten Abschnitt der Drahthülle 2 bereitstellt.

Alternativ kann der Aufweitungsabschnitt 2b durch ein separates Aufweitungshülsenstück 6 gebildet sein, das geeignet mit einer angrenzenden Komponente der Drahthülle 2 verbunden wird. Die Figuren 1 bis 3, 5, 6 und 8 bis 21 zeigen Beispiele mit dem separaten Aufweitungshülsenstück 6. Dabei ist das Aufweitungshülsenstück 6 an einem proximalen Endbereich 7 mit einem distalen Endbereich 8 des proximalen Hüllabschnitts 2a direkt oder über ein Verbindungshülsenstück 9 verbunden. Bei den gezeigten Beispielen der Figuren 5, 6, 10 bis 13, 18, 20 und 21 ist das Verbindungshülsenstück 9 verwendet, bei den Beispielen der Figuren 1 bis 3, 8, 9, 14 bis 17 und 19 ist eine direkte Verbindung des proximalen Endbereichs 7 des Aufweitungshülsenstücks 6 mit dem distalen Endbereich 8 des proximalen Hüllabschnitts 2a gewählt. Weiter ist das separate Aufweitungshülsenstück 6 am distalen Endbereich 3 des Aufweitungsabschnitts 2b mit einem proximalen Endbereich 10 des distalen Hüllabschnitts 2c, soweit dieser vorhanden ist, direkt oder über ein Verbindungshülsenstück 11 verbunden. Die Beispiele der Figuren 5, 6, 10 bis 13, 18, 20 und 21 verwenden die Verbindungshülsenstücke 9, 11, bei den Ausführungsbeispielen der Figuren 1 bis 3, 8, 9, 14 bis 17 und 19 ist das Aufweitungshülsenstück 6 an seinem distalen Endbereich 3 direkt mit dem proximalen Endbereich 10 des distalen Hüllabschnitts 2c verbunden.

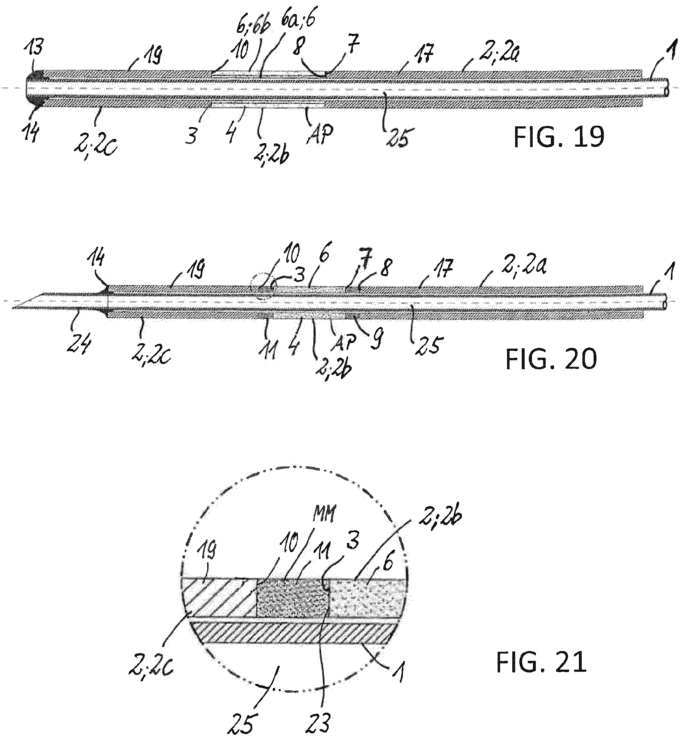

Das jeweilige Verbindungshülsenstück 9, 11 kann je nach Bedarf und Anwendungsfall aus einem Metallmaterial oder einem Kunststoffmaterial bestehen. Dies gilt insbesondere auch für die gezeigten Ausführungen, die das betreffende Verbindungshülsenstück 9, 11 enthalten. Vorteilhaft kann es hierbei sein, das Aufweitungshülsenstück 6 und das oder die Verbindungshülsenstücke 9, 11 jeweils aus Kunststoffmaterial zu fertigen und dabei für das jeweilige Verbindungshülsenstück 9, 11 ein härteres, weniger elastisches Kunststoffmaterial zu verwenden als für das Aufweitungshülsenstück 6. Das ermöglicht es, das jeweilige Verbindungshülsenstück 9, 11 durch Verschmelzen seines härteren Kunststoffmaterials mit dem weicheren Kunststoffmaterial des Aufweitungshülsenstücks 6 fest und zuverlässig mit dem angrenzenden Aufweitungshülsenstück 6 zu verbinden. Stellvertretend ist in Fig. 21 eine derartige Schmelzverbindung 23 explizit markiert, mit welcher der Aufweitungsabschnitt 2b bzw. das Aufweitungshülsenstück 6 an seinem distalen Endbereich 3 fest mit dem proximalen Stirnende des Verbindungshülsenstücks 11 durch Verschmelzen des weicheren Kunststoffmaterials des Aufweitungshülsenstücks 6 mit dem härteren Kunststoffmaterial des Verbindungshülsenstücks 11 verbunden ist. Dies beugt dem unerwünschten Effekt vor, dass sich bei hohen Druckbelastungen während des Aufweitens des Aufweitungshülsenstücks 6 das elastischere bzw. weichere Kunststoffmaterial des Aufweitungshülsenstücks 6 unbeabsichtigt über das härtere bzw. weniger elastische Metall- oder Kunststoffmaterial des Verbindungshülsenstücks 9, 11 stülpt.

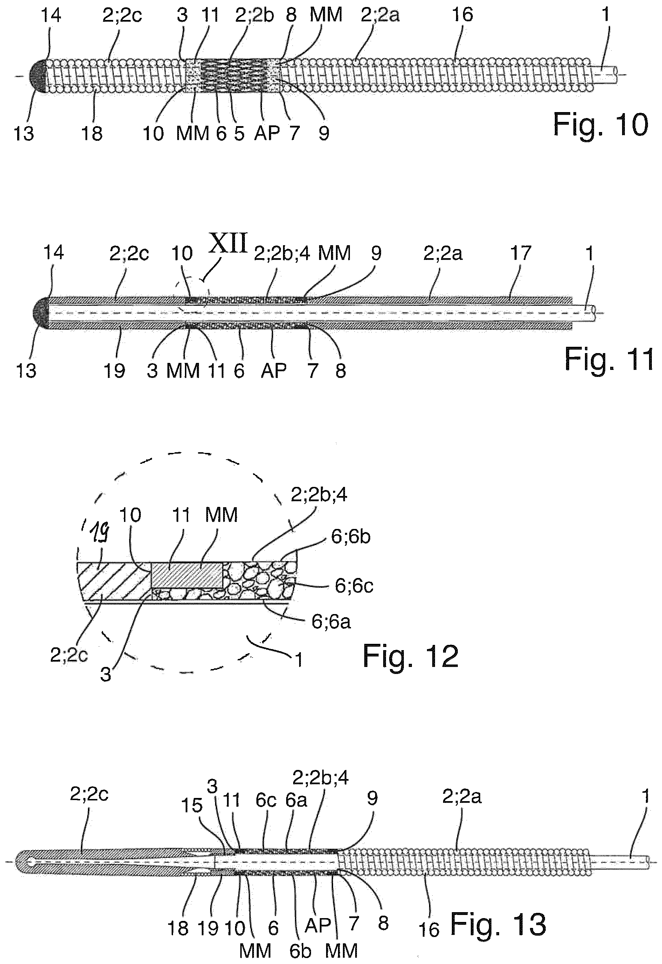

In entsprechenden Ausführungen erstreckt sich das jeweilige Verbindungshülsenstück 9, 11, das radial außen mit dem Aufweitungshülsenstück 6 im Wesentlichen fluchtet, radial nach innen nicht mit der Wanddicke des Aufweitungshülsenstücks 6 bis zum Kerndraht 1, sondern besitzt eine demgegenüber geringere Hülsenwanddicke, so dass zwischen dem betreffenden Verbindungshülsenstück 9, 11 und dem Kerndraht 1 ein axialer Endbereich des Aufweitungshülsenstücks 6 mit geringerer Wanddicke als der übrige Bereich des Aufweitungshülsenstücks 6 verbleibt. Dies ist in Fig. 12 erkennbar, die zusammen mit der Fig. 11 ein solches Ausführungsbeispiel zeigt. In alternativen Ausführungen besitzen das jeweilige Verbindungshülsenstück 9, 11 und das Aufweitungshülsenstück 6 gleiche Wanddicken, und das jeweilige Verbindungshülsenstück 9, 11 grenzt nur mit seinem betreffenden Stirnende mit Berührkontakt an das zugeordnete Stirnende des Aufweitungshülsenstücks 6 an. Dies ist z.B. für die Ausführung der Fig. 20 der Fall, wie aus der Detailansicht von Fig. 21 ersichtlich.

Die Verwendung des Aufweitungshülsenstücks 6 ermöglicht u.a. eine materialtechnische Entkopplung des Aufweitungsabschnitts 2b von der übrigen Drahthülle 2. So kann beispielsweise der Aufweitungsabschnitt 2b in Form des Aufweitungshülsenstücks 6 aus einem elastischeren Material, wie einer Nickel-Titan-Legierung, insbesondere Nitinol, oder einem anderen superelastischen Material, und der proximale und/oder der distale Hüllabschnitt 2a, 2c aus einem Material mit höherer Festigkeit gebildet sein, wie einem Edelstahlmaterial.

Bei den Ausführungsbeispielen der Figuren 1 bis 4, 7, 8 und 14 bis 17 kann, wie oben erwähnt, je nach Bedarf und Anwendungsfall und je nach Material und Struktur des Aufweitungsabschnitts 2b der Aufweitungsabschnitt 2b einteilig mit dem proximalen Hüllabschnitt 2a und/oder mit dem distalen Hüllabschnitt 2c oder durch das separate Aufweitungshülsenstück 6 gebildet sein. So kann insbesondere beim Ausführungsbeispiel von Fig. 4 der Aufweitungsabschnitt 2b einteilig mit dem proximalen und dem distalen Hüllabschnitt 2a, 2c gefertigt sein, indem das in diesem Fall verwendete Vollwandmaterial der Drahthülle 2 im Bereich des Aufweitungsabschnitts 2b in eine perforierte Struktur umgewandelt wird, z.B. durch Laserschneiden.

In entsprechenden Ausführungsformen ist der Aufweitungsabschnitt 2b mehrlagig mit mindestens einer Innenlage 6a und einer Außenlage 6b aufgebaut, wobei sich mindestens zwei der mehreren Lagen 6a, 6b in ihrem Material und/oder ihrer Struktur und/oder ihren physikalischen Eigenschaften, z.B. ihren Gleiteigenschaften, unterscheiden. Die Fig. 6, 7, 9, 11 bis 13 und 18 zeigen derartige Ausführungsbeispiele mit mehrlagigem Aufweitungsabschnitt 2b. Speziell besitzen die Drahtinstrumente der Fig. 6, 7 und 9 einen zweilagigen Aufbau des Aufweitungsabschnitts 2b, bei dem folglich die Innenlage 6a und die Außenlage 6b aneinander angrenzen. Bei den Ausführungsbeispielen der Fig. 11 bis 13 und 18 besitzt der Aufweitungsabschnitt 2b drei Lagen.

Die mehrlagige Ausführung des Aufweitungsabschnitts 2b erlaubt es, dessen radiale Außenseite und dessen radiale Innenseite materialtechnisch zu entkoppeln. So kann beispielsweise, wie im Fall von Fig. 6, die Innenlage 6a aus einem durchlässigen Gewebematerial und die Außenlage 6b aus einem fluiddicht umhüllenden Material, wie einem vergleichsweise biegeweichen Kunststoffmaterial, bestehen. Die Innenlage 6a und die Außenlage 6b können sich in insbesondere auch ihren Gleiteigenschaften unterscheiden.

Der mehrlagige Aufweitungsabschnitt 2b kann vollständig durch das separate Aufweitungshülsenstück 6 gebildet sein. Es ist auch möglich, alle oder zumindest eine der Lagen einteilig mit dem proximalen Hüllabschnitt 2a und/oder mit dem distalen Hüllabschnitt 2c der Drahthülle 2 zu bilden.

In entsprechenden Ausführungen sind die Innenlage 6a und die Außenlage 6b des mehrlagigen Aufweitungsabschnitts 2b jeweils fluiddicht ausgeführt, und der Aufweitungsabschnitt 2b beinhaltet darüber hinaus eine Zwischenlage 6c aus einem flüssigen oder gelartigen Material radial zwischen der Innenlage 6a und der Außenlage 6b, wie dies bei den Ausführungen mit dementsprechend dreilagigem Aufweitungsabschnitt 2b gemäß den Fig. 11 bis 13 und 18 der Fall ist.

In entsprechenden Realisierungen ist die Außenlage 6b des Aufweitungsabschnitts 2b von einem fluiddichten Hüllschlauch 12 gebildet, der sich entweder nur über die Länge der Innenlage 6a hinweg erstreckt oder sich über die Länge der Innenlage 6a und über zumindest einen Teil der Länge des proximalen Hüllabschnitts 2a und/oder des distalen Hüllabschnitts 2c hinweg erstreckt. Die Fig. 7 veranschaulicht ein zugehöriges Ausführungsbeispiel, bei dem sich der Hüllschlauch 12 über die Länge der hier exemplarisch aus einem perforierten Material bestehenden Innenlage 6a und über die gesamte Länge des distalen Hüllabschnitts 2c sowie über einen an den Aufweitungsabschnitt 2b angrenzenden Teil der Länge des proximalen Hüllabschnitts 2a hinweg erstreckt. Die perforierte Innenlage 6a kann z.B. wie der perforierte Aufweitungsabschnitt 2b im Beispiel von Fig. 4 einteilig mit dem proximalen und dem distalen Hüllabschnitt 2a, 2c durch Laserschneiden aus dem Vollwandmaterial der Drahthülle 2 gefertigt sein. Der Hüllschlauch 12 kann mit gewünschten Eigenschaften gefertigt sein, z.B. aus einem hochelastischen Material und/oder aus einem Material mit guten Gleiteigenschaften, d.h. mit hoher Gleitfähigkeit.

In Ausführungen, die zur Verwendung unter Röntgenstrahlungsanwendungen und/oder Magnetresonanzanwendungen gedacht sind, ist es von Vorteil, wenn der Aufweitungsabschnitt 2b und/oder der proximale Hüllabschnitt 2a und/oder der distale Hüllabschnitt 2c, soweit vorhanden, und/oder das oder die Verbindungshülsenstücke 9, 11 zum Anbringen des separaten Aufweitungshülsenstücks 6, soweit vorhanden, ein Röntgenstrahlungs-sichtbares und/oder ein MR-sichtbares Markermaterial MM enthält. Dies kann bei allen gezeigten Ausführungsbeispielen vorgesehen sein. Explizit angegeben ist exemplarisch zu den Ausführungsbeispielen der Fig. 5, 6, 10 bis 13, 18, 20 und 21 das Vorhandensein des Markermaterials MM in den dortigen Verbindungshülsenstücken 9, 11. Dabei kann es sich beispielsweise in den Fällen der Fig. 5, 6, 10 bis 13 und 18 optional um ein Platin- oder Wolframmaterial handeln, aus dem das betreffende Verbindungshülsenstück 9, 11 in diesem Fall gebildet sein kann, oder das Markermaterial MM kann, wie für das Ausführungsbeispiel der Fig. 20 und 21 möglich, z.B. in Form von Markerpartikeln in ein Kunststoffmaterial des betreffenden Verbindungshülsenstücks 9, 11 eingebettet oder oberflächlich an diesem angebracht sein.

In entsprechenden Ausführungen ist der Kerndraht 1 über eine distale Endkuppe 13 mit einem distalen Endbereich 14 des distalen Hüllabschnitts 2c verbunden, wie dies bei den Ausführungsbeispielen der Fig. 1 bis 7, 9 bis 12 und 14 bis 17 und 19 der Fall ist. In alternativen, nicht gezeigten Ausführungen ist der Kerndraht 1 über die distale Endkuppe 13 direkt mit dem distalen Endbereich 3 des Aufweitungsabschnitts 2b verbunden. In anderen Ausführungen, wie im Beispiel von Fig. 20, schließt das Drahtinstrument distal mit einem Kanülenstück 24 ab, wie es für entsprechende Anwendungen erwünscht ist. Die Endkuppe 13 bzw. das Kanülenstück 24 ist z.B. durch Verkleben oder Laserschweißen fest am distalen Endbereich 14 des distalen Hüllabschnitts 2c angebracht.

In entsprechenden Realisierungen ist der Kerndraht 1 über eine umfangsseitige Verbindung 15, d.h. eine Verbindung, die sich in einem zugehörigen axialen Teilbereich des Kerndrahts 1 an dessen Außenumfang befindet, mit einem umgebenden Bereich des distalen Hüllabschnitts 2c verbunden. Die Fig. 8, 13 und 18 zeigen zugehörige Ausführungsbeispiele. In alternativen, nicht gezeigten Realisierungen ist der Kerndraht 1 über die umfangsseitige Verbindung 15 direkt mit dem distalen Endbereich 3 des Aufweitungsabschnitts 2b verbunden.

In entsprechenden Ausführungsformen ist der proximale Hüllabschnitt 2a zumindest bereichsweise durch ein Schraubenfederstück 16 oder ein Hülsenstück 17 aus massivem Hülsenwandmaterial, d.h. einem Vollwandmaterial, gebildet. Die Fig. 10, 13 und 18 zeigen Ausführungen mit dem Schraubenfederstück 16 im proximalen Hüllabschnitt 2a, die Fig. 1 bis 9, 11, 12, 14 bis 17, 20 und 21 zeigen Ausführungsbeispiele mit dem massiven Hülsenstück 17 für den proximalen Hüllabschnitt 2a.

In entsprechenden Ausführungen ist der distale Hüllabschnitt 2c zumindest bereichsweise durch ein Schraubenfederstück 18 oder ein Hülsenstück 19 aus massivem Hülsenwandmaterial gebildet. Die Fig. 8, 10, 13 und 18 zeigen Ausführungsbeispiele mit dem Schraubenfederstück 18 im distalen Hüllabschnitt 2c. Bei den Ausführungsbeispielen der Fig. 1 bis 9,11 bis 13 bis 21 beinhaltet der distale Hüllabschnitt 2c das Hülsenstück 19 aus massivem Hülsenwandmaterial. Wie anhand der Beispiele in den Fig. 8, 13 und 18 für den distalen Hüllabschnitt 2c ersichtlich, kann der proximale Hüllabschnitt 2a und/oder der distale Hüllabschnitt 2c auch kombiniert ein Schraubenfederstück und ein Hülsenstück aus massivem Hülsenwandmaterial aufweisen. Des Weiteren kann, wie in den Beispielen der Fig. 8, 13 und 18, der Kerndraht 1 in seinem distalen Endbereich in das Material des distalen Hüllabschnitts 2c eingebettet sein, z.B. einschließlich eines verdickten distalen Kerndrahtendes, was zu einer hohen Festigkeit der Verbindung des Kerndrahtes 1 mit dem distalen Hüllabschnitt 2c der Drahthülle 2 beitragen kann.

In vorteilhaften Ausführungsformen beinhaltet das Drahtinstrument eine proximale, nutzerbetätigbare Bedieneinheit 20 zur axialen Verschiebung des Kerndrahts 1 gegenüber dem proximalen Hüllabschnitt 2a. Für die Bedieneinheit 20 lässt sich eine beliebige herkömmliche Bedieneinheit verwenden, mit der die gewünschte axiale Relativbewegung von Kerndraht 1 und proximalem Hüllabschnitt 2a der Drahthülle 2 bewirkt werden kann. Die Bedieneinheit 20 ist daher nur in Fig. 1 und nur schematisch in Blockdiagrammform und stellvertretend für die anderen gezeigten Ausführungsbeispiele angegeben.

In vorteilhaften Ausführungsformen bilden der Kerndraht 1 und die Drahthülle 2 einen endoskopischen Führungsdraht. Bei allen gezeigten Ausführungsbeispielen lässt sich der Aufbau aus Kerndraht 1 und Drahthülle 2 als ein solcher Führungsdraht verwenden. Für diese Anwendung als Führungsdraht besitzt der Aufbau aus Kerndraht 1 und Drahthülle 2 typischerweise einen Außendurchmesser im Bereich von höchstens wenigen Millimetern und vorzugsweise im Bereich kleiner als 1 mm.

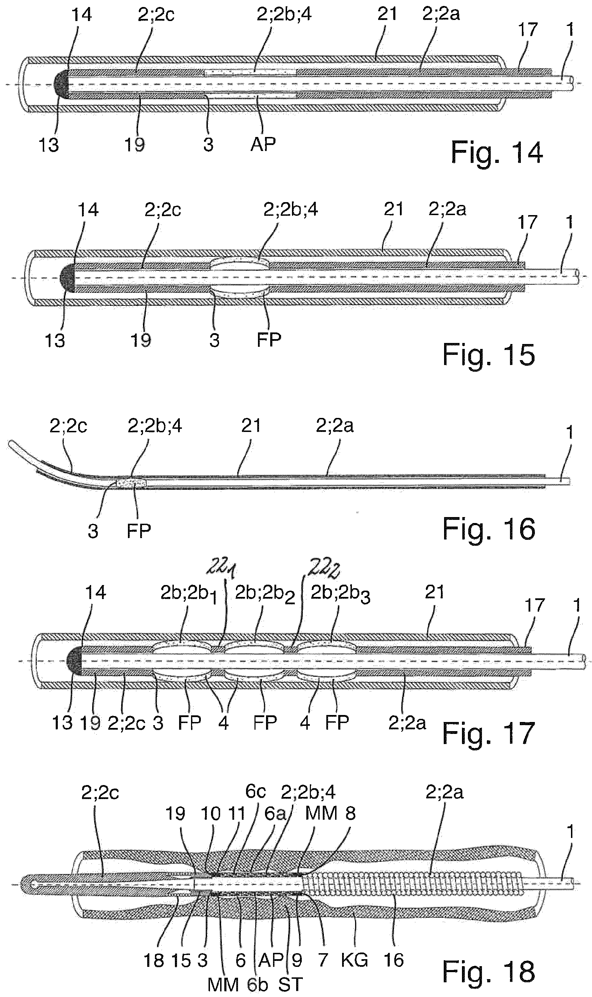

Fig. 18 veranschaulicht konkret die Verwendung eines solchen Führungsdrahts in der Ausführung von Fig. 13 zum Dehnen bzw. Aufweiten einer Stenose ST in einem Körpergewebekanal KG. Dazu wird das Drahtinstrument bzw. der Führungsdraht mit dem Aufweitungsabschnitt 2b in seiner nicht aufgeweiteten Ausgangsposition AP in den Körpergewebekanal KG eingeführt und vorgeschoben, bis der Aufweitungsabschnitt 2b des Führungsdrahts im Bereich der Stenose ST zu liegen kommt. Sobald der Aufweitungsabschnitt 2b im Bereich der Stenose ST liegt, wie in Fig. 18 gezeigt, kann der Benutzer das Instrument über die Bedieneinheit 20 am proximalen Endbereich des Instruments betätigen und dadurch die axiale Relativbewegung von Kerndraht 1 und Drahthülle 2 bewirken, die dazu führt, dass sich der Aufweitungsabschnitt 2b in seine radial nach außen aufgeweitete Funktionsposition FP verformt. Dadurch dehnt der Aufweitungsabschnitt 2b die Stenose ST auf. Nach erfolgtem Aufdehnen hebt der Benutzer die axiale Stauchkraft auf den Aufweitungsabschnitt 2b auf, indem er die entgegengesetzte axiale Relativbewegung von Kerndraht 1 und Drahthülle 2 vornimmt bzw. freigibt, wodurch sich der Aufweitungsabschnitt 2b aufgrund seiner Elastizität von seiner aufgeweiteten Funktionsposition FP wieder in seine nicht aufgeweitete Ausgangsposition AP zurückverformt.

In den Ausführungsformen der Fig. 1 bis 18 ist der Kerndraht 1 aus einem massiven Metall- oder Kunststoffmaterial gebildet, d.h. aus einem Vollmaterial. In alternativen Ausführungen, wie sie in den Fig. 19 bis 21 gezeigt sind, ist der Kerndraht 1 aus einem hohlen Metall- oder Kunststoffmaterial gebildet und stellt dadurch mit seinem hohlen Inneren ein Lumen 25 bereit, das bei Bedarf in bestimmter Weise genutzt werden kann, z.B. als ein fluid- oder gasführender Kanal und/oder dazu, einen Führungsdraht oder einen anderen stabförmigen Körper durch das Innere des hohlen Kerndrahtes hindurchzuführen. So kann in diesem Fall das Drahtinstrument z.B. dadurch in einen Körpergewebekanal eingeführt werden, dass es über einen zuvor in den Körpergewebekanal eingeführten Führungsdraht aufgeschoben wird, wobei der Führungsdraht im Lumen 25 des hohlen Kerndrahtes 1 aufgenommen wird.

Bei einer weiteren möglichen Anwendung kann das Drahtinstrument mit seinem radial aufweitbaren Aufweitungsabschnitt 2b, wenn dieser fluiddicht ausgeführt ist, dazu benutzt werden, den Blutfluss in einer Vene oder einer Arterie temporär zu stoppen.

In einer weiteren Anwendung des Drahtinstruments lässt sich der radial aufweitbare Aufweitungsabschnitt 2b als Vakuumstempel oder Druckstempel bei einem endoskopischen Instrument benutzen. In diesem Fall kann es zweckmäßig sein, den Aufweitungsabschnitt 2b an seiner Oberfläche geeignet zu beschichten, z.B. mit einem PTFE(Polytetrafluorethylen)-Material.

In vorteilhaften Ausführungsformen beinhaltet das Drahtinstrument einen Instrumentenschlauch 21, der den Kerndraht 1 und die Drahthülle 2 wenigstens im Bereich des proximalen Hüllabschnitts 2a und des Aufweitungsabschnitts 2b umgibt. Speziell umgibt der Instrumentenschlauch 21 den proximalen Hüllabschnitt 2a und den Aufweitungsabschnitt 2b, wenn sich dieser in seiner Ausgangsposition AP befindet, mit radialem Abstand. Wenn sich der Aufweitungsabschnitt 2b in seiner Funktionsposition FP befindet, legt er sich axial bewegungskoppelnd radial gegen den Instrumentenschlauch 21 an, d.h. gegen dessen Innenwand. Die Fig. 14, 15 und 17 veranschaulichen derartige Ausführungsbeispiele.

Bei diesen Ausführungen kann folglich durch das radiale Aufweiten des Aufweitungsabschnitts 2b der Aufbau aus Kerndraht 1 und Drahthülle 2 mit dem umgebenden Instrumentenschlauch 21 so gekoppelt werden, dass die gekoppelten Komponenten gegen axiale Relativbewegung gesichert sind. Zusätzlich kann durch das Anlegen bzw. Andrücken des sich radial aufweitenden Aufdehnungsabschnitts 2b gegen die Innenwand des Instrumentenschlauchs 21 bei Bedarf eine Abdichtung des Innenraums des Instrumentenschlauchs 21 in Richtung proximal nach hinten bereitgestellt werden.

In vorteilhaften Ausführungen ist das mit dem Instrumentenschlauch 21 ausgerüstete Drahtinstrument ein endoskopisches Katheterinstrument, dessen Katheterschlauch vom Instrumentenschlauch 21 gebildet ist. Die Ausführungsbeispiele der Fig. 14, 15 und 18 lassen sich in dieser Weise bei Bedarf als Katheterinstrument verwenden.

Fig. 16 veranschaulicht die Anwendung der axialen Bewegungskopplung von Kerndraht 1 und Drahthülle 2 einerseits, d.h. dem dadurch gebildeten Führungsdraht, und dem umgebenden Instrumentenschlauch 21 andererseits durch den expandierbaren aufweitenden Aufweitungsabschnitt 2b zum problemlosen Vorschieben des Instrumentenschlauchs 21 in einen Körpergewebekanal. Dazu wird der Führungsdraht nicht ganz in der benötigten Länge in den Körpergewebekanal vorgeschoben, bevor der Instrumentenschlauch 21 auf den Führungsdraht aufgeschoben wird. Vielmehr wird zuvor der Instrumentenschlauch 21 auf den z.B. nur über eine relativ kleine Länge in den Körpergewebekanal eingeführten Führungsdraht aufgeschoben, solange dies problemlos möglich ist, und danach wird der Aufweitungsabschnitt 2b von seiner Ausgangsposition AP in seine Funktionsposition FP verbracht, wodurch der Instrumentenschlauch 21 am Führungsdraht quasi festgeklemmt wird, so dass er zusammen mit dem Führungsdraht weiter in den Körpergewebekanal vorgeschoben werden kann bzw. von diesem in der Vorbewegung mitgenommen wird. Dadurch lässt sich vermeiden, dass es zu Auffaltungen des Instrumentenschlauchs 21 über dem Führungsdraht kommt, wie dies auftreten kann, wenn der Führungsdraht zunächst vollständig vorgeschoben und danach der Instrumentenschlauch 21 auf den Führungsdraht aufgeschoben wird und es beim Aufschieben des Instrumentenschlauchs 21 auf den Führungsdraht zu Problemen kommt, z.B. bei einer relativ starken Krümmung des Führungsdrahts in einem gewissen Abschnitt oder bei schlechten Gleitbedingungen des Instrumentenschlauchs 21 am Führungsdraht, hier speziell der Drahthülle 2.

Wie die gezeigten und die weiteren oben erläuterten Ausführungsbeispiele deutlich machen, stellt die Erfindung in vorteilhafter Weise ein Drahtinstrument zur Verfügung, das sich insbesondere zur Verwendung in der medizinischen Endoskopietechnik eignet, speziell für entsprechende medizinische Führungsdrähte und Funktionsschlauchinstrumente, wie Katheter.

Je nach Bedarf kann das Drahtinstrument zusätzlich zu dem Aufweitungsabschnitt der Drahthülle mit einem distalen Nutzelement ausgerüstet sein, das eine zugehörige Nutzfunktion erfüllt. Das Nutzelement kann beispielsweise ein entfaltbarer Drahtkorb oder Filter, z.B. zum Einfangen und/oder Entfernen von steinartigen Ablagerungen bzw. Gebilden wie Nierensteine oder von Blutgerinnseln zum Beseitigen bzw. Verhindern von Thrombosen, eine Schere, eine Zange, eine Kamera oder andere optische Komponente oder ein distaler Führungsdraht- oder Katheterabschnitt sein, der durch variables, gesteuertes axiales Bewegen bzw. Biegen oder Krümmen eine zugehörige Nutzfunktion erfüllt.

Wie der Fachmann anhand der vorliegenden Erfindungsoffenbarung erkennt, kann das Instrument außer für endoskopische Zwecke auch für anderweitige medizinische und nicht-medizinische Zwecke eingesetzt werden, in denen Bedarf an einem Drahtinstrument besteht, dessen einen Kerndraht umgebende Drahthülle einen gesteuert radial elastisch aufweitbaren Abschnitt besitzt.

## Patentansprüche

1. Drahtinstrument, insbesondere endoskopisches Drahtinstrument, mit
- einem Kerndraht (1) und
- einer den Kerndraht (1) entlang zumindest eines Teils seiner Länge umgebenden Drahthülle (2), wobei der Kerndraht (1) gegenüber einem proximalen Hüllabschnitt (2a) der Drahthülle (2) axial relativbeweglich angeordnet ist, **dadurch gekennzeichnet, dass**
- die Drahthülle (2) einen an den proximalen Hüllabschnitt (2a) distal anschließenden Aufweitungsabschnitt (2b) aufweist, der biegeweicher als der proximale Hüllabschnitt (2a) ist und dafür eingerichtet ist, sich unter Einwirkung einer axialen Stauchkraft axial verkürzend von einer Ausgangsposition (AP) in eine Funktionsposition (FP) radial elastisch aufzuweiten, und
- der Kerndraht (1) mit einem distalen Endbereich (3) des Aufweitungsabschnitts (2b) axial bewegungsgekoppelt verbunden ist.

2. Drahtinstrument nach Anspruch 1, weiter **dadurch gekennzeichnet, dass** die Drahthülle (2) einen an den Aufweitungsabschnitt (2b) distal anschließenden distalen Hüllabschnitt (2c) beinhaltet, der biegesteifer als der Aufweitungsabschnitt (2b) ist und mit dem der Kerndraht (1) axial bewegungsgekoppelt verbunden ist.

3. Drahtinstrument nach Anspruch 1 oder 2, weiter **dadurch gekennzeichnet, dass** der Aufweitungsabschnitt (2b) mehrere axial hintereinanderliegende Aufweitungsteilabschnitte (2b₁, 2b₂, 2b₃) umfasst, von denen jeder biegeweicher als der proximale Hüllabschnitt (2a) ist und dafür eingerichtet ist, sich unter Einwirkung der axialen Stauchkraft radial elastisch aufzuweiten.

4. Drahtinstrument nach einem der Ansprüche 1 bis 3, weiter **dadurch gekennzeichnet, dass** der Aufweitungsabschnitt (2b) einen fluiddichten Abschnitt (4) der Drahthülle (2) und/oder oder einen perforierten Abschnitt (5) der Drahthülle (2) beinhaltet.

5. Drahtinstrument nach einem der Ansprüche 1 bis 4, weiter **dadurch gekennzeichnet, dass** der Aufweitungsabschnitt (2b) einteilig mit dem proximalen Hüllabschnitt (2a) und/oder mit dem distalen Hüllabschnitt (2c) gebildet ist oder durch ein separates Aufweitungshülsenstück (6) gebildet ist, das an einem proximalen Endbereich (7) mit einem distalen Endbereich (8) des proximalen Hüllabschnitts (2a) direkt oder über ein Verbindungshülsenstück (9) verbunden ist und/oder an dem distalen Endbereich (3) mit einem proximalen Endbereich (10) des distalen Hüllabschnitts (2c) direkt oder über ein Verbindungshülsenstück (11) verbunden ist.

6. Drahtinstrument nach einem der Ansprüche 1 bis 5, weiter **dadurch gekennzeichnet, dass** der Aufweitungsabschnitt (2b) mehrlagig mit mindestens einer Innenlage (6a) und einer Außenlage (6b) aufgebaut ist, wobei sich mindestens zwei der mehreren Lagen in ihrem Material und/oder ihrer Struktur und/oder ihren physikalischen Eigenschaften unterscheiden.

7. Drahtinstrument nach Anspruch 6, weiter **dadurch gekennzeichnet, dass** die Innenlage (6a) und die Außenlage (6b) jeweils fluiddicht ausgeführt sind und der Aufweitungsabschnitt (2b) eine Zwischenlage (6c) aus einem flüssigen oder gelartigen Material zwischen der Innenlage (6a) und der Außenlage (6b) aufweist.

8. Drahtinstrument nach Anspruch 6 oder 7, weiter **dadurch gekennzeichnet, dass** die Außenlage (6b) von einem fluiddichten Hüllschlauch (12) gebildet ist, der sich nur über die Länge der Innenlage (6a) hinweg erstreckt oder der sich über die Länge der Innenlage (6a) und über zumindest einen Teil der Länge des proximalen Hüllabschnitts (2a) und/oder des distalen Hüllabschnitts (2c) hinweg erstreckt.

9. Drahtinstrument nach einem der Ansprüche 1 bis 8, weiter **dadurch gekennzeichnet, dass** der Aufweitungsabschnitt (2b) und/oder der proximale Hüllabschnitt (2a) und/oder der distale Hüllabschnitt (2c) und/oder das jeweilige Verbindungshülsenstück (9, 11) ein Röntgenstrahlungs-sichtbares und/oder ein Magnetresonanz(MR)-sichtbares Markermaterial (MM) enthält.

10. Drahtinstrument nach einem der Ansprüche 1 bis 9, weiter **dadurch gekennzeichnet, dass**
- der Kerndraht (1) über eine distale Endkuppe (13) direkt mit dem distalen Endbereich (3) des Aufweitungsabschnitts (2b) oder mit einem distalen Endbereich (14) des distalen Hüllabschnitts (2c) verbunden ist und/oder
- der Kerndraht (1) über eine umfangsseitige Verbindung (15) direkt mit dem distalen Endbereich (3) des Aufweitungsabschnitts (2b) oder mit einem umgebenden Bereich des distalen Hüllabschnitts (2c) verbunden ist.

11. Drahtinstrument nach einem der Ansprüche 1 bis 10, weiter **dadurch gekennzeichnet, dass**
- der proximale Hüllabschnitt (2a) zumindest bereichsweise durch ein Schraubenfederstück (16) oder ein Hülsenstück (17) aus massivem Hülsenwandmaterial gebildet ist und/oder
- der distale Hüllabschnitt (2c) zumindest bereichsweise durch ein Schraubenfederstück (18) oder ein Hülsenstück (19) aus massivem Hülsenwandmaterial gebildet ist.

12. Drahtinstrument nach einem der Ansprüche 1 bis 11, weiter **dadurch gekennzeichnet, dass**
- der Kerndraht (1) und die Drahthülle (2) einen endoskopischen Führungsdraht bilden oder
- der Kerndraht (1) als Hohldraht aus einem hohlen Metall- oder Kunststoffmaterial gebildet ist.

13. Drahtinstrument nach einem der Ansprüche 1 bis 12, weiter **gekennzeichnet durch** eine proximale, nutzerbetätigbare Bedieneinheit (20) zur axialen Verschiebung des Kerndrahtes (1) gegenüber dem proximalen Hüllabschnitt (2a).

14. Drahtinstrument nach Anspruch 12 oder 13, weiter **gekennzeichnet durch** einen Instrumentenschlauch (21), der den Kerndraht (1) und die Drahthülle (2) wenigstens im Bereich des proximalen Hüllabschnitts (2a) und des Aufweitungsabschnitts (2b) umgibt, wobei der Instrumentenschlauch (21) den proximalen Hüllabschnitt (2a) und den Aufweitungsabschnitt (2b) in dessen Ausgangsposition (AP) mit radialem Abstand umgibt und sich der Aufweitungsabschnitt (2b) in seiner Funktionsposition (FP) axial bewegungskoppelnd radial gegen den Instrumentenschlauch (21) anlegt.

15. Drahtinstrument nach Anspruch 14, weiter **dadurch gekennzeichnet, dass** es ein endoskopisches Katheterinstrument ist, wobei der Instrumentenschlauch (21) einen Katheterschlauch bildet.
